# EUROPÄISCHE PATENTSCHRIFT

(11) **EP 3 725 718 B1**
(45) Veröffentlichungstag und Bekanntmachung des Hinweises auf die Patenterteilung: **08.09.2021**
(21) Anmeldenummer: 19170163.0
(22) Anmeldetag: 18.04.2019
(51) Int. Cl.: B65H 54/10, B65B 63/04

(54) **WICKELKOPF FÜR CHIRURGISCHES NAHTMATERIAL**
WINDING HEAD FOR SURGICAL SUTURES
TÊTE D'ENROULEMENT POUR MATÉRIEL CHIRURGICAL DE SUTURE

(43) Veröffentlichungstag der Anmeldung: 21.10.2020
(73) Patentinhaber: Harro Höfliger Verpackungsmaschinen GmbH, 71573 Allmersbach im Tal (DE)
(72) Erfinder: Neff, Ingmar, 71573 Allmersbach im Tal (DE)
(74) Vertreter: Schmid, Barbara

(56) Entgegenhaltungen:
- US-A- 5 012 413
- US-A1- 2002 069 617
- US-A1- 2016 317 148

## Beschreibung

### TECHNISCHES GEBIET

Die Erfindung betrifft einen Wickelkopf für chirurgisches Nahtmaterial. Mit einem solchen Wickelkopf wird ein Faden des chirurgischen Nahtmaterials in eine Packung gewickelt. Aus dieser Packung wird das chirurgische Nahtmaterial zu einem späteren Zeitpunkt zur Verwendung beispielsweise während einer Operation entnommen.

### STAND DER TECHNIK

Vorrichtungen zum Wickeln von chirurgischem Nahtmaterial in eine Packung sind bekannt. Die bekannten Vorrichtungen weisen in der Regel einen Wickelkopf auf, durch den das eigentliche Positionieren des Fadens an chirurgischem Nahtmaterial in der Packung erfolgt. Die Wickelköpfe sind regelmäßig an die Form der Packung angelehnt und weisen an ihrem Randbereich ein umlaufendes Führungssystem auf. An dem Führungssystem ist ein Führungswagen mit einem Befestigungsarm verschieblich gelagert. Der Befestigungsarm kann an der hierfür vorgesehenen Wickelstation statisch angebunden sein. Bei dieser Wickelstation kann es sich beispielsweise um eine Station an einem Rundschalttisch handeln. Der Führungswagen fährt in einer umlaufenden Richtung um das Führungssystem herum, so dass sich das Führungssystem rotierend bewegt und dabei das chirurgische Nahtmaterial in die Packung wickelt.

Insbesondere bei ovalen Packungen besitzt auch der Wickelkopf und damit das Führungssystem eine ovale Gestalt (s. z.B. US 2016/0317148). Das Führungssystem besitzt daher regelmäßig zwei halbkreisförmige Außenbereiche, die bevorzugt durch zwei parallel verlaufende gerade Strecken miteinander verbunden sind. An dem Übergang zwischen den geraden Strecken und den halbkreisförmigen Außenbereichen kommt es verstärkt zu Verschleiß in Form von Abrieb und Überbelastung der Führungsrollen des Führungswagens. Dieser Verschleiß führt zu Spiel in der Führung, was die exakte Positionierung des chirurgischen Nahtmaterials verhindert oder zumindest erschwert, so dass nur noch geringere Umdrehungszahlen möglich sind. Der Wickelkopf muss daher verhältnismäßig häufig ausgetauscht werden, was zum Stillstand der entsprechenden Vorrichtung und zu erheblichem Wartungsaufwand führt.

### DARSTELLUNG DER ERFINDUNG

Ausgehend von diesem vorbekannten Stand der Technik liegt der Erfindung die Aufgabe zugrunde, einen verbesserten Wickelkopf für chirurgisches Nahtmaterial anzugeben, bei dem der Verschleiß während des Betriebs möglichst gering ist, so dass lange Standzeiten bei hohen Umdrehungszahlen erreicht werden können.

Der erfindungsgemäße Wickelkopf ist durch die Merkmale des Hauptanspruchs 1 gegeben. Sinnvolle Weiterbildungen der Erfindung sind Gegenstand von sich an diesen Anspruch anschließenden weiteren Ansprüchen.

Der erfindungsgemäße Wickelkopf besitzt einen Befestigungsarm sowie einen Führungswagen, der an dem Befestigungsarm gelagert ist. Darüber hinaus besitzt der Wickelkopf ein umlaufendes Führungssystem, das verschieblich an dem Führungswagen gelagert ist. Bei der Rotation des Führungssystems wird das chirurgische Nahtmaterial geführt in eine Packung eingelegt. Das Führungssystem besitzt zwei voneinander beabstandete erste Bahnen, die durch zwei zweite Bahnen, die jeweils etwa halbkreisförmig ausgebildet sind, miteinander verbunden sind. Insgesamt ergibt sich dadurch eine etwa ovale Form des Führungssystems. Erfindungsgemäß ist in zumindest einem der insgesamt vier Übergangsbereiche zwischen einer ersten und einer zweiten Bahn eine Übergangskurve vorhanden, die die erste und die zweite Bahn miteinander verbindet.

Durch die Veränderung des Übergangs von einer ersten, etwa gerade verlaufenden Bahn zu einer zweiten Bahn, die als gebogene Strecke ausgebildet ist, durch eine solche Übergangskurve kommt es im Bereich des Übergangs nicht länger zu einem sprunghaften Anstieg der Querbeschleunigung. Vielmehr handelt es sich nunmehr um eine stetige Bewegung, so dass Belastungsspitzen auf die Rollenführungen des Führungswagens und auf das Führungssystem vermindert werden können. Dies führt zu einer größeren Laufruhe und einer gesteigerten Laufleistung, so dass größere Wickelgeschwindigkeiten möglich werden.

Die größten Belastungen treten in Laufrichtung des Führungswagens gesehen bei einem Übergang von einer etwa gerade verlaufenden ersten Bahn auf eine etwa halbkreisförmige zweite Bahn auf. Vorzugsweise kann daher zumindest einer dieser Übergänge - vorzugsweise beide Übergänge - mit einer solchen Übergangskurve ausgestattet sein.

Sofern der Wickelkopf eine Wickelbewegung sowohl im Uhrzeigersinn als auch im Gegen-Uhrzeigersinn erlauben soll, können vorzugsweise in allen vier Übergangsbereichen entsprechende Übergangskurven vorgesehen sein.

Die zumindest eine Übergangskurve kann vorzugsweise als Klothoide, als Blosskurve oder als Kurve höherer Ordnung ausgebildet sein, so dass die Bewegung nicht sprunghaft, sondern vielmehr nahezu stetig geändert wird. Insbesondere Klothoiden lassen sich mathematisch exakt berechnen und erlauben somit einen besonders weichen und verschleißfreien Übergang.

Die beiden ersten Bahnen können insbesondere gerade ausgebildet sein. In diesem Fall können die beiden ersten Bahnen etwa parallel zueinander verlaufen. Die beiden ersten Bahnen können jedoch auch leicht gebogen verlaufen und damit einen sehr großen Radius aufweisen. Der Radius der beiden ersten Bahnen wäre in diesem Fall deutlich größer als der Radium der beiden zweiten Bahnen. Dabei müssen die beiden ersten Bahnen nicht zwingend denselben Radius aufweisen. Auch die beiden zweiten Bahnen können jeweils unterschiedliche Radien aufweisen.

Erfindungsgemäss soll der Führungswagen zumindest eine innere Führungsrolle und zumindest eine äußere Führungsrolle aufweisen, zwischen denen das Führungssystem verschieblich gelagert ist. Zumindest eine der Führungsrollen soll dabei federnd gelagert sein. Die federnde Lagerung dieser zumindest einen Führungsrolle sorgt dafür, dass eine automatische und permanente Einstellung der Führungsvorspannung zwischen den Führungsrollen erreicht werden kann. Dadurch kann der Wartungsaufwand reduziert werden. Gleichzeitig können die Werte der Führungsvorspannung unabhängig von der Erfahrung und dem Fingerspitzengefühl des jeweiligen Monteurs eingestellt werden. Durch die zumindest eine gefederte Führungsrolle kann der Wickelkopf darüber hinaus toleranter gegenüber Formabweichungen des Führungssystems sein, die durch Fertigungstoleranzen hervorgerufen werden können. Insgesamt kann somit der Verschleiß am Wickelkopf weiter reduziert werden.

Weitere Vorteile und Merkmale der Erfindung sind den in den Ansprüchen ferner angegebenen Merkmalen sowie den nachstehenden Ausführungsbeispielen zu entnehmen.

### KURZE BESCHREIBUNG DER ZEICHNUNG

Die Erfindung wird im Folgenden anhand der in der Zeichnung dargestellten Ausführungsbeispiele näher beschrieben und erläutert. Es zeigen:
- Fig. 1: eine Seitenansicht des an einem Bauteil befestigten Wickelkopfes mit Befestigungsarm mit Führungswagen,
- Fig. 2: eine perspektivische Ansicht des Wickelkopfes gemäß Fig. 1,
- Fig. 3: eine Draufsicht auf die Unterseite des Führungssystems des Wickelkopfes gemäß Fig. 1 und 2, und
- Fig. 4: eine schematische Draufsicht auf die Unterseite des Führungswagens mit den Führungsrollen.

### WEGE ZUM AUSFÜHREN DER ERFINDUNG

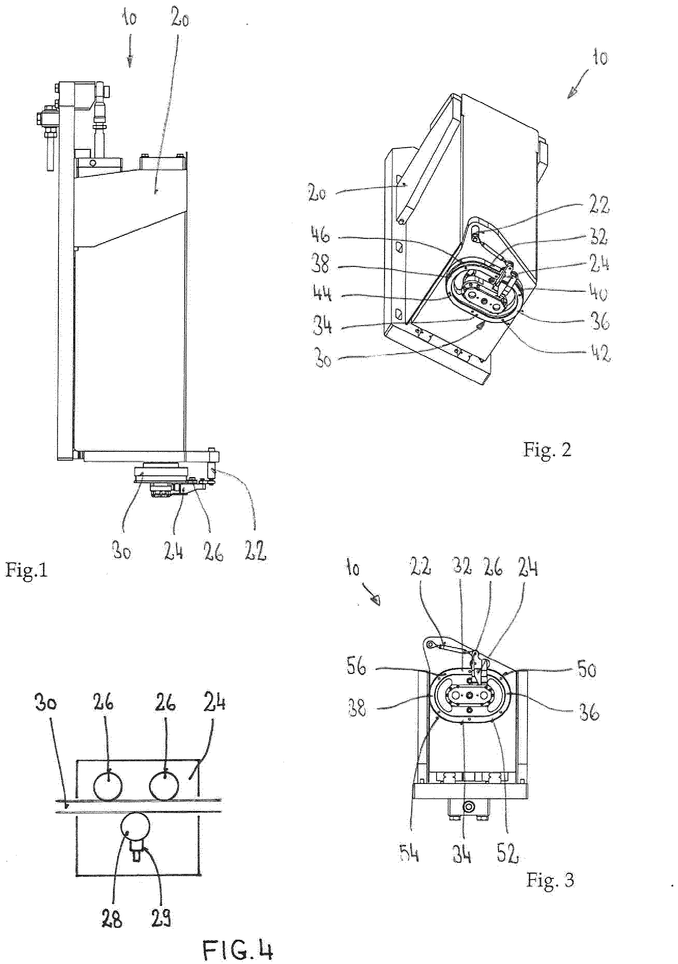

Der erfindungsgemäße Wickelkopf 10 für chirurgisches Nahtmaterial ist in Fig. 1 bis 3 dargestellt. An einem Bauteil 20 ist ein Befestigungsarm 22 beweglich gelagert. Am Ende des Befestigungsarms 22 ist ein Führungswagen 24 gelagert. Der Führungswagen 24 besitzt im vorliegenden Beispielsfall mehrere Führungsrollen 26. Mit diesen Führungsrollen 26 ist der Führungswagen 24 an einem umlaufenden Führungssystem 30 verschieblich gelagert.

Der Führungswagen 24 (siehe insbesondere Fig. 4) weist im vorliegenden Beispielsfall zwei äußere Führungsrollen 26 und eine innere Führungsrolle 28 auf. Das Führungssystem 30 ist zwischen den äußeren Führungsrollen 26 und der inneren Führungsrolle 28 verschieblich gelagert. Im vorliegenden Beispielsfall ist die innere Führungsrolle 28 über eine Federung 29 an dem Führungswagen befestigt, so dass diese federnd gelagert ist. Dadurch kann eine automatische und permanente Einstellung der Führungsvorspannung zwischen den Führungsrollen 26, 28 erreicht werden kann. Dies kann den Wartungsaufwand der Führungsrollen 26, 28 und damit auch des Führungswagens 24 deutlich reduzieren. Darüber hinaus ist der Wickelkopf auf diese Weise toleranter gegenüber Formabweichungen des Führungssystems, die durch Fertigungstoleranzen entstehen können.

Das umlaufende Führungssystem 30 (siehe insbesondere Fig. 3) weist zwei erste Bahnen 32, 34 auf, die im vorliegenden Beispielsfall gerade ausgebildet sind und parallel zueinander verlaufen. Die beiden ersten Bahnen 32, 34 werden durch zwei etwa halbkreisförmige zweite Bahnen 36, 38 miteinander verbunden. Insgesamt ergibt sich dadurch eine etwa ovale Form des Führungssystems 30.

Das Führungssystem 30 ist in der Regel in seiner Form an die Außenkontur derjenigen Packung angepasst, in die das zu wickelnde chirurgische Nahtmaterial eingelegt werden soll. Abhängig von der Außenkontor dieser Packung können die beiden ersten Bahnen 32, 34 auch nicht parallel zueinander verlaufen und / oder nicht gerade, sondern vielmehr in einem großen Krümmungsradius gebogen verlaufen. Auch müssen die beiden zweiten Bahnen 36, 38 nicht jeweils identische Radien aufweisen.

Durch die Form des Führungssystems 30 mit zwei ersten Bahnen 32, 34 und zwei zweiten Bahnen 36, 38 ergeben sich insgesamt vier Übergangsbereiche 40, 42, 44, 46. Würden die ersten Bahnen 32, 34 und die zweiten Bahnen 36, 38 in diesen Übergangsbereichen 40, 42, 44, 46 ohne weitere Maßnahmen aneinander grenzen, so würde eine sprunghafte Änderung der Bewegung (von gerade auf gekrümmt beziehungsweise von gekrümmt auf gerade) erfolgen. Dies führt zu einem erhöhten Verschleiß der Führungsrollen 26, 28 des Führungswagens 24, so dass der Führungswagen 24 regelmäßig gewartet und ausgetauscht werden muss. Darüber hinaus ist auch das Führungssystem 30 selbst in diesen Übergangsbereichen 40, 42, 44, 46 einem erhöhten Abrieb ausgesetzt, so dass auch hier ein regelmäßiger Austausch erfolgen sollte.

Im vorliegenden Beispielsfall ist daher im Bereich aller vier Übergangsbereiche 40, 42, 44, 46 jeweils eine Übergangskurve 50, 52, 54, 56 vorgesehen. Die Übergangskurven 50, 52, 54, 56 weisen keinen konstanten Radius auf, vielmehr ändert sich die Krümmung der Übergangskurven 50, 52, 54, 56 über deren gesamten Verlauf, so dass ein weicher Übergang der ersten Bahnen 32, 34 auf die zweiten Bahnen 36, 38 und umgekehrt möglich ist. Dies ermöglicht eine nahezu stetige Änderung der Bewegung, die besonders schonend für die Führungsrollen 26, 28 des Führungswagens 24 und auch für das Führungssystem 30 ist.

Der Verschleiß an den Führungsrollen 26, 28 ist an den Übergangsbereichen 40, 44 am größten, an denen eine gerade Bahn 32, 34 in eine gekrümmte Bahn 36, 38 übergeht. Daher sollte insbesondere an diesen Übergangsbereichen 40, 44 eine Übergangskurve 50, 54 vorgesehen werden. Sofern eine Rotation im Uhrzeigersinn und im Gegen-Uhrzeigersinn möglich sein soll, sollte an allen vier Übergangsbereichen 40, 42, 44, 46 eine entsprechende Übergangskurve 50, 52, 54 56 vorgesehen werden.

## Patentansprüche

1. Wickelkopf (10) für chirurgisches Nahtmaterial
- mit einem Befestigungsarm (22),
- mit einem Führungswagen (24), der an dem Befestigungsarm (22) gelagert ist,
- mit einem umlaufenden Führungssystem (30), das verschieblich an dem Führungswagen (24) gelagert ist,
- wobei das umlaufende Führungssystem (30) zwei voneinander beabstandete erste Bahnen (32, 34) besitzt,
- wobei die beiden ersten Bahnen (32, 34) des Führungssystems (30) durch zwei zweite Bahnen (36, 38), die etwa halbkreisförmig ausgebildet sind, miteinander verbunden sind,
- wobei in zumindest einem der Übergangsbereiche (40,42, 44, 46) zwischen einer ersten Bahn (32, 34) und einer zweiten Bahn (36, 38) eine Übergangskurve (50, 52, 54, 56) vorhanden ist, die die erste Bahn (32, 34) und die zweite Bahn (36, 38) miteinander verbindet,
- wobei der Führungswagen (24) zumindest eine innere Führungsrolle (28) und zumindest eine äußere Führungsrolle (26) aufweist, zwischen denen das Führungssystem (30) verschieblich gelagert ist, **dadurch gekennzeichnet, dass**
- zumindest eine der Führungsrollen (28) federnd gelagert ist.

2. Wickelkopf nach Anspruch 1,
- **dadurch gekennzeichnet, dass**
- die zumindest eine Übergangskurve (50,54) in einem der Übergangsbereiche (40, 44) von einer ersten Bahn (32, 34) auf eine zweite Bahn (46, 48) angeordnet ist.

3. Wickelkopf nach Anspruch 2,
- **dadurch gekennzeichnet, dass**
- in allen vier Übergangsbereichen (40, 42, 44, 46) zwischen einer ersten Bahn (32, 34) und einer zweiten Bahn (36, 38) eine Übergangskurve (50, 52, 54, 56) vorhanden ist, die die erste Bahn (32, 34) und die zweite Bahn (36, 38) miteinander verbindet.

4. Wickelkopf nach einem der vorstehenden Ansprüche,
- **dadurch gekennzeichnet, dass**
- die zumindest eine Übergangskurve (50,52,54,56) als Klothoide, als Blosskurve oder als Kurve höherer Ordnung ausgebildet ist.

5. Wickelkopf nach einem der vorstehenden Ansprüche,
- **dadurch gekennzeichnet, dass**
- die beiden ersten Bahnen (32, 34) etwa parallel zueinander verlaufen.

## Claims

1. Winding head (10) for surgical suture material,
- with a securing arm (22),
- with a guide carriage (24), which is mounted on the securing arm (22),
- with a circumferential guide system (30), which is mounted displaceably on the guide carriage (24),
- wherein the circumferential guide system (30) has two first tracks (32, 34) which are spaced apart from each other,
- wherein the two first tracks (32, 34) of the guide system (30) are connected to each other by two second tracks (36, 38), which are of approximately semi-circular shape,
- wherein a transition curve (50, 52, 54, 56) is present in at least one of the transition regions (40, 42, 44, 46) between a first track (32, 34) and a second track (36, 38), which transition curve (50, 52, 54, 56) connects the first track (32, 34) and the second track (36, 38) to each other,
- wherein
the guide carriage (24) has at least one inner guide roller (28) and at least one outer guide roller (26), between which the guide system (30) is mounted displaceably, **characterized in that**
- at least one of the guide rollers (28) is mounted resiliently.

2. Winding head according to Claim 1,
- **characterized in that**
- the at least one transition curve (50, 54) is arranged in one of the transition regions (40, 44) from a first track (32, 34) to a second track (46, 48) .

3. Winding head according to Claim 2,
- **characterized in that**,
- in all four transition regions (40, 42, 44, 46) between a first track (32, 34) and a second track (36, 38), a transition curve (50, 52, 54, 56) is present which connects the first track (32, 34) and the second track (36, 38) to each other.

4. Winding head according to one of the preceding claims,
- **characterized in that**
- the at least one transition curve (50, 52, 54, 56) is configured as a clothoid, as a Bloss curve or as a higher-order curve.

5. Winding head according to one of the preceding claims,
- **characterized in that**
- the two first tracks (32, 34) run approximately parallel to each other.

## Revendications

1. Tête d'enroulement (10) pour matériel chirurgical de suture, comprenant
- un bras de fixation (22),
- un chariot de guidage (24) qui est monté sur le bras de fixation (22),
- un système de guidage périphérique (30) qui est monté coulissant sur le chariot de guidage (24),
- le système de guidage périphérique (30) possédant deux premières bandes (32, 34) espacées l'une de l'autre,
- les deux premières bandes (32, 34) du système de guidage (30) étant reliées ensemble par deux deuxièmes bandes (36, 38) qui sont réalisées approximativement en forme de demi-cercle,
- dans laquelle, dans au moins l'une des zones de transition (40, 42, 44, 46) entre une première bande (32, 34) et une deuxième bande (36, 38), une courbe de transition (50, 52, 54, 56) est présente qui relie la première bande (32, 34) et la deuxième bande (36, 38) l'une à l'autre,
- le chariot de guidage (24) présentant au moins un rouleau de guidage intérieur (28) et au moins un rouleau de guidage extérieur (26) entre lesquels le système de guidage (30) est monté coulissant, **caractérisée en ce que**
- au moins l'un des rouleaux de guidage (28) est monté sur ressort.

2. Tête d'enroulement selon la revendication 1,
- **caractérisée en ce que**
- ladite au moins une courbe de transition (50, 54) est disposée dans l'une des zones de transition (40, 44) d'une première bande (32, 34) à une deuxième bande (46, 48).

3. Tête d'enroulement selon la revendication 2,
- **caractérisée en ce que**
- dans l'ensemble des quatre zones de transition (40, 42, 44, 46), entre une première bande (32, 34) et une deuxième bande (36, 38), une courbe de transition (50, 52, 54, 56) est présente qui relie la première bande (32, 34) et la deuxième bande (36, 38) l'une à l'autre.

4. Tête d'enroulement selon l'une quelconque des revendications précédentes,
- **caractérisée en ce que**
- ladite au moins une courbe de transition (50, 52, 54, 56) est réalisée sous forme de clothoïde, de courbe de Bloss ou de courbe d'ordre supérieur.

5. Tête d'enroulement selon l'une quelconque des revendications précédentes,
- **caractérisée en ce que**
- les deux premières bandes (32, 34) s'étendent approximativement en parallèle l'une à l'autre.
